(19) Europäisches Patentamt
European Patent Office
Office européen des brevets

(11) **EP 1 202 996 B1**

(12) # FASCICULE DE BREVET EUROPEEN

(45) Date de publication et mention de la délivrance du brevet:
**01.10.2003 Bulletin 2003/40**

(21) Numéro de dépôt: **00949690.2**

(22) Date de dépôt: **11.07.2000**

(51) Int Cl.[7]: **C07F 7/00**, A61K 31/00, A61K 6/00

(86) Numéro de dépôt international:
**PCT/FR00/01994**

(87) Numéro de publication internationale:
**WO 01/005797 (25.01.2001 Gazette 2001/04)**

(54) **COMPOSES DE TITANE, LEUR PREPARATION ET UTILISATION**

TITANVERBINDUNGEN, DEREN HERSTELLUNG UND VERWENDUNG

TITANIUM DERIVED COMPOUNDS, PREPARATION AND USE THEREOF

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **16.07.1999 FR 9909194**

(43) Date de publication de la demande:
**08.05.2002 Bulletin 2002/19**

(73) Titulaire: **SANOFI-SYNTHELABO**
**75013 Paris (FR)**

(72) Inventeur: **FINIDORI, Claudine**
**F-92120 Montrouge (FR)**

(74) Mandataire: **Monain, Patrice**
**Sanofi-Synthelabo,**
**174 avenue de France**
**75013 Paris (FR)**

(56) Documents cités:
- **DEAN, PHILIP A. W. ET AL: "Spectroscopic studies of inorganic fluoro complexes. III. fluorine-19nuclear magnetic resonance studies of silicon (IV), germanium(IV), andtitanium(IV) fluoro complexes" J. CHEM. SOC. A (1970), (15), 2569-74, XP002131802**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

L'invention se rapporte à de nouveaux composés dérivés de titane, ainsi qu'à leur préparation.

L'invention se rapporte également à des compositions à usage buccal comprenant de tels composés dérivés de titane.

Il est connu que le tétrafluorure de titane ($TiF_4$) peut se fixer à la surface de la dent, en formant une couche protectrice amorphe, encore appelée glacis, à la surface de l'émail de la dent.

La formation d'une telle couche protectrice à la surface de la dent a conduit à envisager l'utilisation du tétrafluorure de titane comme agent de prévention et de traitement de la carie dentaire.

Le tétrafluorure de titane présente cependant l'inconvénient de présenter une forte acidité en solution aqueuse (pH de l'ordre de 1,5) agressive pour les tissus minéralisés et non compatible avec les pH physiologiques.

Son utilisation dans la prévention de la carie dentaire a donc été limitée jusqu'à présent à un usage professionnel, avec un temps d'application très court, suivi d'un rinçage.

Le tétrafluorure de titane présente de plus l'inconvénient d'être relativement instable, notamment en solution aqueuse.

L'invention a pour but de remédier à ces inconvénients, en proposant des composés dérivés de titane IV comportant du fluor, capables de former un glacis à la surface de la dent, utilisables en solution aqueuse et à des pH physiologiques variant d'environ 6,5 à environ 7,5.

Les composés dérivés de titane selon l'invention répondent à la formule (I) suivante :

$$[TiF_xL_y]^{z-} \qquad (I)$$

Le document J. Chem. Soc. (1970), (15), 2569-74 décrit des complexes fluorés inorganiques de silicium, de titane ou de germanium. Les spectres R. M. N. de différents exemples de ces complexes sont en particulier décrits. dans laquelle L représente un composé de formule (II) suivante :

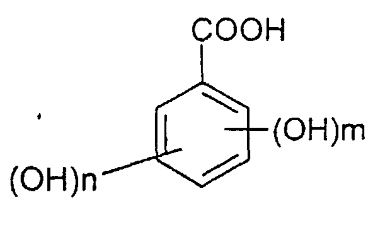

(II)

dans laquelle m est 0 ou 1 et n est 0, 1 ou 2,
et x représente 2, 4 ou 5, y représente 1 ou 2 et z représente 0, 1 ou 2.

Les composés de formule (I) peuvent comporter un ou plusieurs atomes de carbone asymétrique. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de la présente invention peuvent exister sous forme de bases libres ou de sels d'addition à des acides pharmaceutiquement acceptables. Toutes ces formes font partie de l'invention.

Selon l'invention, des composés L utilisables sont notamment les dérivés d'acide benzoïque, notamment l'acide hydroxy-2-benzoïque de formule:

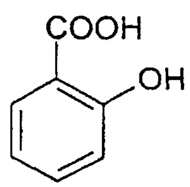

, l'acide hydroxy-3-benzoïque de formule :

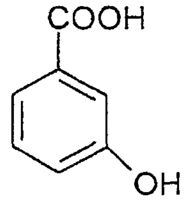

, l'acide hydroxy-4-benzoïque de formule :

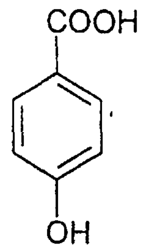

, l'acide dihydroxy-2,3-benzoïque de formule :

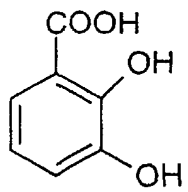

, l'acide dihydroxy-3,4-benzoïque de formule :

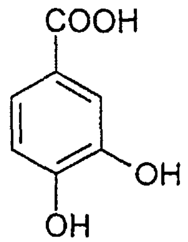

Un exemple de composé dérivé de titane selon l'invention est le composé représenté par la formule suivante (III) :

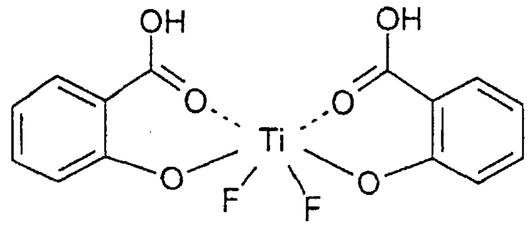

(III)

Les composés dérivés de titane (IV) de l'invention peuvent être préparés en faisant réagir à température ambiante le fluorure de titane IV solide avec une solution d'acide benzoïque dans un solvant anhydre tel que l'acétonitrile, sous atmosphère d'azote.

Les produits de départ sont disponibles dans le commerce ou décrits dans la littérature, ou peuvent être préparés par des méthodes qui y sont décrites ou qui sont connues de l'homme du métier.

L'invention vise également à protéger des compositions à usage buccal comprenant au moins un composé selon l'invention ainsi que l'utilisation des composés de dérivés de titane selon l'invention, en tant qu'agent de protection contre la carie dentaire.

Les compositions de l'invention comprennent au moins un composé selon l'invention en une quantité équivalente à d'environ 10 ppm à environ 10 000 ppm de fluor, calculé à partir du poids moléculaire du composé selon l'invention.

Les compositions de l'invention peuvent se présenter sous les diverses formes usuelles pour les compositions à usage buccal et notamment sous forme de pâte ou gel dentifrice, de bain de bouche, de spray, de mousse, de gargarisme, de gel dentaire ou de gomme à mâcher, baume, pâte, vernis, pastille, comprimé, collutoire, poudre, solution concentrée ou non, le véhicule étant alors choisi selon la forme d'utilisation désirée.

Les compositions de l'invention peuvent contenir, en plus du ou des composés dérivés de titane, à titre de véhicule ou pour leur activité propre, des excipients ou ingrédients habituellement utilisés dans les compositions à usage buccal.

Les compositions de l'invention sont préparées selon les procédés usuels correspondant aux véhicules choisis. Le véhicule physiologiquement acceptable peut être de nature différente selon la forme choisie pour la composition : solution aqueuse, solution hydroalcoolique épaissie ou non, gomme, excipient pâteux ou solide, etc.

Selon les formes désirées, les compositions de l'invention peuvent comprendre en outre au moins un agent de polissage dans des proportions allant jusqu'à 80 % en poids par rapport au poids total de la composition. Les agents de polissage sont par exemple d'origine minérale ou organique. Leur nature peut différer selon le véhicule utilisé pour la forme choisie.

Des agents de polissage utilisables comprennent le carbonate ou bicarbonate de calcium, de magnésium, de sodium, les phosphates et sulfates de calcium, l'alumine et l'alumine hydratée, les silices, les oxydes, hydroxydes, trisilicates et pyrophosphates de magnésium ou de calcium, ou des composés cellulosiques obtenus par broyage de graines de céréales par exemple.

Lorsque les compositions sont sous forme de pâte dentifrice, elles peuvent contenir un agent de polissage dans des proportions généralement comprises entre environ 2 % et environ 70 % en poids, de préférence entre environ 15 % et environ 25 % en poids. Il s'agit en général d'un agent de polissage abrasif minéral constitué par un ou plusieurs composés, en grande partie insolubles dans l'eau. On peut citer à titre d'exemples les métaphosphates de sodium ou de potassium, le phosphate de calcium dihydraté, le phosphate dicalcique, le phosphate tricalcique, le pyrophosphate de calcium, l'alumine, les alumines hydratées et en particulier trihydratées, les silices, les silicates d'aluminium ou de zirconium, la bentonite ainsi que l'orthophosphate de magnésium ou le phosphate trimagnésien, les carbonates et bicarbonates de calcium, de sodium.

Les compositions de l'invention sous forme de pâte dentifrice peuvent également comprendre un ou plusieurs agents de cohésion. De tels agents de cohésion peuvent être incorporés dans des proportions allant jusqu'à environ 10 % en poids par rapport au poids total de la composition, et de préférence entre environ 0,5 % et environ 3 % en poids. Les agents de cohésion peuvent être choisis notamment parmi les épaississants naturels tels que les alginates et les pectines, les gommes naturelles comme la gomme adragante, les gommes de xanthane, de guar, de caroube ou de carraghénane, ou les épaississants synthétiques tels que les dérivés de cellulose, comme le sel de sodium de la carboxyméthylcellulose, la méthycellulose, les hydroxyalkylcelluloses, les acides polyacryliques réticulés, les carraghénates synthétiques.

Selon certaines formes de réalisation, les compositions de l'invention peuvent comprendre un ou plusieurs agents tensioactifs suffisamment stables et moussants. Les agents tensioactifs utilisables peuvent être de nature anionique, amphotère, zwitterionique, cationique ou non-ionique.

De façon générale, les agents tensioactifs sont présents dans les compositions de l'invention dans une gamme pondérale variant d'environ 0,01 % à environ 4 %, de préférence d'environ 0,1 % à environ 2 %, par rapport au poids total de la composition.

En outre, les compositions de l'invention peuvent comprendre d'autres agents actifs utilisés dans l'hygiène buccale, notamment des agents connus pour réduire la mauvaise haleine, tels que par exemple les cyclodextrines, ou des composés du zinc tels que par exemple les halogénures de zinc, l'acétate de zinc, le citrate de zinc ou le fluorure de zinc, la chlorhexedine, le chlorure de cétylpyridinium.

Les compositions de l'invention peuvent comprendre également divers agents de cohésion, agents épaississants, agents antibiotiques, agents édulcorants, humectants ou rafraîchissants, agents peptisants, agents conservateurs, agents sucrants, colorants, arômes, substances et agents aromatisants, agents plastifiants, agents antibactériens ou bactéricides, vitamines, agents anti-tartre, cicatrisants, vasomoteurs, agents anti-saignements, agents actifs sur la gencive, agents anti-inflammatoires tels que l'enoloxone, la benzydamine, l'allantoïne, le permethol, etc.

Ces différents agents sont présents dans les compositions de l'invention selon la forme d'utilisation.

Ainsi, lorsque la composition à usage buccal est un spray, le véhicule peut être une solution hydro-alcoolique et la composition peut comprendre en outre des arômes, des agents peptisants, des agents édulcorants, humectants ou rafraîchissants.

Lorsque la composition à usage buccal est sous forme de bain de bouche, le véhicule peut être non aqueux, aqueux ou hydroalcoolique avec un ou plusieurs agents tensioactifs et/ou un ou plusieurs agents épaississants, et peut comprendre en outre des agents bactéricides, des édulcorants et des substances aromatisantes.

A titre d'exemple, lorsque la composition est sous forme de gel dentaire, elle peut en outre comprendre des agents actifs sur la gencive.

Lorsque la composition est sous forme de gomme à mâcher, elle comprend au moins une gomme masticable naturelle ou synthétique, et peut comprendre de plus des agents plastifiants, des vitamines, des agents aromatisants ou de sapidité, des agents sucrants, des agents humectants, des bactéricides, des agents conservateurs, des colorants.

Parmi les gommes masticables utilisables figurent notamment le latex d'hévéa, la gomme chicle, la gomme schlong, l'acétate de polyvinyle et les élastomères de synthèse, notamment caoutchouc silicone, caoutchouc butyle ainsi que leurs dérivés et/ou mélanges.

Les compositions à usage buccal de l'invention peuvent comprendre en outre un agent édulcorant, Parmi les agents édulcorants utilisables, on peut citer le saccharose, le lactose, le fructose, le xylitol, le cyclamate de sodium, le maltose ou le saccharinate de sodium, les glycyrrhizinates de sodium ou d'ammonium, les mélanges d'alpha-glucosyl/stéviolglucoside, le D-mannitol, l'aspartame, l'acesulfam K, le sorbitol, le lycosin et leurs mélanges.

Les agents édulcorants sont présents généralement en une quantité allant jusqu'à environ 2 % en poids par rapport au poids total de la composition.

Comme agents humectants, on peut citer le sorbitol, le glycérol ou le xylitol, présents à ce titre dans des concentrations pouvant atteindre 70 % en poids.

En outre, des agents rafraîchissants, tels que le menthol ou l'éthylmaltol peuvent être incorporés dans les compositions de l'invention.

Il est également possible d'utiliser des agents conservateurs, choisis parmi notamment le parahydroxybenzoate de méthyle, le parahydroxybenzoate de propyle, le benzoate de sodium, la chlorhexedine, à des concentrations généralement d'environ 1 % en poids ou inférieures.

Les substances aromatisantes utilisables comprennent toutes celles autorisées à ce titre dans l'alimentation. Par exemple, on peut citer les essences de menthe, d'anis, d'eucalyptus, de cannelle, de girofle, de sauge, de réglisse, ou de fruits comme l'orange, le citron, la mandarine ou la fraise. Les substances aromatisantes sont généralement présentes en une quantité en poids d'environ 5 % ou moins.

Les compositions de l'invention peuvent également comprendre un agent antibactérien choisi de préférence parmi notamment les huiles essentielles, extraits végétaux ou des substances telles que le chlorure de cétylpyridinium, l'alexidine, l'octinidine, l'hexetidine, le phénoxyéthanol, l'alcool phénéthylique, le triclosan, la chlorhexidine, le chlorure de cétylpyridinium, le delmopinol, dans des proportions allant jusqu'à environ 10 % en poids par rapport au poids total de la composition.

L'exemple qui suit illustre la préparation d'un composé dérivé de titane de l'invention. Les microanalyses élémentaires, et les spectres I. R. et R. M. N. confirment la structure du composé obtenu.

Exemple 1.

Préparation du composé de formule (III) :

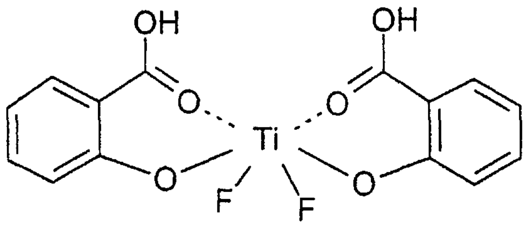

(III)

A 10 g ( 0,072 mole) d'une solution d'acide salicylique dans 100 ml d'acétonitrile anhydre sont ajoutés sous agitation et sous azote 5 g ( 0,040 mole) de fluorure de titane solide. Le mélange réactionnel prend rapidement une couleur orange, et la solution est laissée sous agitation pendant 24 heures. Après décantation et concentration par évaporation, la solution est refroidie pendant la nuit et de petits cristaux en aiguilles se forment, correspondant à l'acide salicylique n'ayant pas réagi. La solution est filtrée à nouveau, concentrée et refroidie à 4°C pour donner de petits cristaux monocliniques jaunes-orangés. Après filtration et séchage sous vide, le composé de formule (III) est obtenu avec un rendement d'environ 49 %. Point de fusion : 157-160°C (décomposition)

Les composés de l'invention ont fait l'objet d'études biologiques qui ont mis en évidence leurs propriétés de formation d'un glacis à la surface de la dent et leur intérêt comme substances pour le traitement ou la prévention de la carie dentaire.

Un échantillon frais de dent est déposé dans une ampoule de verre contenant du coton humidifié et du thymol, à une température de + 4°C.

L'échantillon est débarrassé des tissus mous éventuellement présents sur la dent. La dent est ensuite polie dans une coupelle en caoutchouc contenant de la pierre ponce exempte de fluorure, puis rincée dans un bain ultrasonique. L'échantillon est traité avec une solution aqueuse à pH 5 du composé (III) de l'invention pendant 10 minutes à 37°C, L'échantillon est ensuite lavé à l'eau pendant une minute et une fenêtre pratiquée à la surface de la dent est observée par microscopie.

La formation d'une couche protectrice amorphe sur la surface de la dent après traitement de l'échantillon par le composé (III) est mise en évidence.

Les résultats de ces essais biologiques montrent que les composés de l'invention présentent des propriétés de formation d'un glacis protecteur sur la surface de la dent.

Ils peuvent être utilisés dans le traitement et la prévention de la carie dentaire.

Des exemples de composition de l'invention sont donnés ci-après.

| Formulation pour pâte dentifrice. | |
|---|---|
| Ingrédient | Quantité |
| Composé de titane | qsp 2500 ppm de F |
| Permethol | 0,250 g |
| Sorbitol 70 % | 25,00 g |
| Silices précipitées | 18,00 g |
| Lauryl sulfate de sodium | 2,00 g |
| Carraghénate de sodium | 1,00 g |
| Oxyde de titane | 1,00 g |
| Arôme menthe | 0,950 g |
| Gesweet | 0,100 g |

(suite)

| Formulation pour pâte dentifrice. | |
|---|---|
| Ingrédient | Quantité |
| Parabens | 0,400 g |
| Eau | qsp 100,00 g |

| Formulation pour pâte dentrifrice. | |
|---|---|
| Ingrédient | Quantité |
| Composé de titane | qsp 1 500 ppm de F |
| Digluconate de chlorhexidine | 0,125 g |
| Acétate de vitamine E | 0,5 g |
| Sorbitol 70 % | 28,00 g |
| Silices précipitées | 11,00 g |
| Lauryl sulfate de sodium | 0,750 g |
| Chimexane NF | 0,750 g |
| Carboxyméthylcellulose sodique | 1,300 g |
| Arôme menthe | 1,00 g |
| Saccharinate de sodium | 0,150 g |
| Oxyde de titane | 1,00 g |
| Parabens | 0,300 g |
| Phosphate dodecahydraté | 0,070 g |
| Eau | qsp 100,00 g |

| Formulation pour bain de bouche. | |
|---|---|
| Ingrédient | Quantité |
| Composé de titane | qsp 250 ppm de F |
| Méthyl-4-esculétol de monoéthanolate de sodium | 1 g |
| D-Panthénol | 5 g |
| Crémophor RH 410 | 0,5 g |
| Alcool à 95 % (V/V) | 80 g |
| Gesweet | 1 g |
| Arôme menthe | 0,795 g |
| Bleu patenté | 0,004 g |
| Eau purifié | qsp 1 000 g |

| Formulation pour bain de bouche. | |
|---|---|
| Ingrédient | Quantité |
| Composé de titane | qsp 250 ppm de F |
| Benzoate de sodium | 0,4 g |
| Alcool à 95 % (V/V) | 10,00 g |
| Saccharinate de sodium | 0,005 g |
| Acide benzoïque | 0,100 g |
| Arôme mentholé | 0,035 g |
| Levomenthol | 0,010 g |
| Colorants | 0,0012 g |
| Eau | qsp 100,00 g |

## Revendications

**1.** Composé dérivé de titane répondant à la formula (I) suivante :

$$[TiF_xL_y]^{z-} \quad (I)$$

dans laquelle L représente un composé de formula (II) suivante :

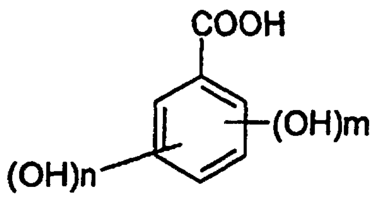

**(II)**

dans laquelle m est 1 et n est 0, 1 ou 2,
et x représente 2, 4 ou 5, y représente 1 ou 2 et z représente 0, 1 ou 2; sous forme d'énantioméres, de diastéréoisomères, ainsi que leurs mélanges, y compris les mélanges racémiques, de bases libres ou de sels pharmaceutiquement acceptables.

**2.** Composé selon la revendication 1, **caractérisé en ce que** les composés L sont choisis parmi les dérivés d'acide benzoïque, notamment l'acide hydroxy-2-benzoïque de formule :

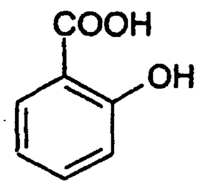

, l'acide hydroxy-3-benzoïque de formule

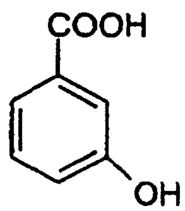

, l'acide hydroxy-4-benzoïque de formule :

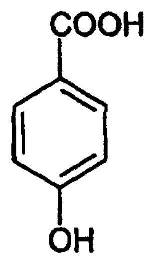

, l'acide dihydroxy-2,3-benzoïque de formule :

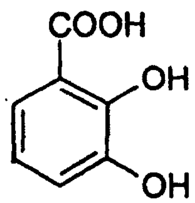

, l'acide dihydroxy-3,4-benzoïque de formule :

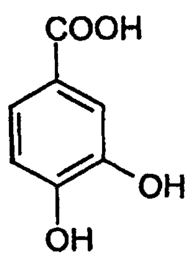

3. Composé selon la revendication 1, **caractérisé en ce qu'**il répond à la formule suivante (III) :

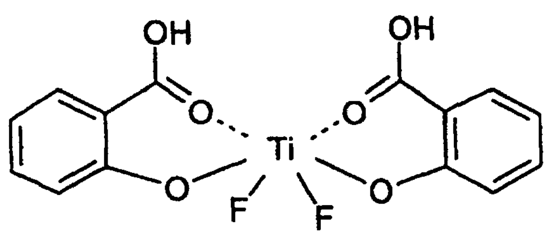

**(III)**

4. Procédé de préparation d'un composé dérivé de titane selon l'une quelconque des revendications 1 à 3, **caractérisé en ce que** l'on fait réagir le fluorure de titane IV solide avec une solution d'acide benzoïque dans un solvant anhydre tel que l'acétonitrile, sous atmosphère d'azote.

5. Composition à usage buccal destinée à lutter contre la carie dentaire, **caractérisée en ce qu'**elle comprend au moins un composé dérivé de titane répondant à la formula (I) suivante :

$$[TiF_xL_y]^{z-} \quad (I)$$

dans laquelle L représente un composé de formule (II) suivante :

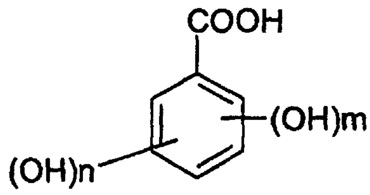

(II)

dans laquelle m est 0, 1 et n est 0, 1 ou 2,
et x représente 2, 4 ou 5, y représente 1 ou 2 et z représente 0, 1 ou 2; sous forme d'énantiomères, de diastéréoisomères, ainsi que leurs mélanges, y compris les mélanges racémiques, de bases libres ou de sels pharmaceutiquement acceptables.

6. Composition à usage buccal selon la revendication 5, **caractérisée en ce qu'**elle comprend au moins un composé dérivé de titane en une quantité équivalente à d'environ 10 ppm à environ 10 000 ppm de fluor.

7. Composition à usage buccal selon la revendication 5 ou 6, **caractérisée en ce qu'**elle se présente sous forme de pâte ou gel dentifrice, de bain de bouche, de spray, de mousse, de gargarisme, de gel dentaire ou de gomme à mâcher, baume, pâte, vernis, pastille, comprimé, collutoire, poudre, solution concentrée ou non.

8. Composition à usage buccal selon l'une quelconque des revendications 5 à 7, **caractérisée en ce qu'**elle comprend en outre au moins un agent de polissage dans des proportions allant jusqu'à 80 % en poids par rapport au poids total de la composition, d'origine minérale ou organique.

9. Composition à usage buccal selon la revendication 8, **caractérisée en ce que** l'agent de polissage comprend notamment le carbonate et bicarbonate de calcium, de magnésium, de sodium, les phosphates et sulfates de calcium, l'alumine et l'alumine hydratée, les silices, les oxydes, hydroxydes, trisilicates et pyrophosphates de magnésium, des composés cellulosiques obtenus par broyage de graines de céréales, les métaphosphates de sodium ou de potassium, le phosphate de calcium dihydraté, le phosphate dicalcique, le phosphate tricalcique, le pyrophosphate de calcium, l'alumine, les alumines hydratées et en particulier trihydratées, les silicates d'aluminium ou de zirconium, la bentonite ainsi que l'orthophosphate de magnésium ou le phosphate trimagnésien.

10. Composition à usage buccal selon l'une quelconque des revendications 5 à 9, **caractérisée en ce qu'**elle comprend en outre un ou plusieurs agents de cohésion, dans des proportions allant jusqu'à environ 10 % en poids par rapport au poids total de la composition, choisis notamment parmi les épaississants naturels tells que les alginates et les pectines, les gommes naturelles comme la gomme adragante, les gommes de xanthane, de guar, de caroube ou de carraghénane, les carraghénates synthétiques ou des épaississants synthétiques tels que les dérivés de cellulose, comme le sel de sodium de la carboxyméthylcellulose, la méthycellulose, les hydroxyalkylcelluloses ou les acides polyacryliques réticulés.

11. Composition à usage buccal selon l'une quelconque des revendications 5 à 10, **caractérisée en ce qu'**elle comprend en outre un ou plusieurs agents tensioactifs de nature anionique, amphotère, zwitterionique, cationique ou non-ionique.

12. Composition à usage buccal selon l'une quelconque des revendications 5 à 11, **caractérisée en ce qu'**elle comprend en outre un ou plusieurs agents actifs utilisés dans l'hygiène buccale, notamment des agents connus pour réduire la mauvaise haleine, tels que par exemple la chlorhexedine, le chlorure de cétylpyridinium, les cyclodextrines ou des composés du zinc tels que les halogénures de zinc, l'acétate de zinc, le citrate de zinc ou le fluorure de zinc.

**13.** Composition à usage buccal selon l'une quelconque des revendications 5 à 12, **caractérisée en ce qu'**elle comprend en outre un ou plusieurs agents de cohésion, agents épaississants, agents antibiotiques, agents édulcorants, humectants ou rafraîchissants, agents peptisants, agents conservateurs, agents sucrants, colorants, arômes, substances et agents aromatisants, agents plastifiants, agents antibactériens ou bactéricides, vitamines, agents anti-tartre, cicatrisants, vasomoteurs, agents anti-saignements, agents actifs sur la gencive, agents anti-inflammatoires tels que l'enoxolone, la benzydamine, l'allantoïne, le permethol.

**14.** Composition à usage buccal selon la revendication 13, **caractérisée en ce que** les agents édulcorants comprennent le saccharose, le lactose, le fructose, le xylitol, le cyclamate de sodium, le maltose ou le saccharinate de sodium, les glycyrrhizinates de sodium ou d'ammonium, les mélanges d'alphaglucosyl/stéviolglucoside, le D-mannitol, l'aspartame, l'acesulfam K, le sorbitol, le lycosin et leurs mélanges.

**15.** Composition à usage buccal selon la revendication 13, **caractérisée en ce que** les agents antibactériens comprennent les huiles essentielles, extraits végétaux ou des substances telles que l'alexidine, l'octinidine, l'héxétidine, le phénoxyéthanol, l'alcool phénéthylique, le triclosan, la chlorhexidine, le chlorure de cétylpyridinium, le delmopinol, dans des proportions allant jusqu'à environ 10 % en poids par rapport au poids total de la composition.

**16.** Composé dérivé de titane répondant à la formule (I) suivante :

$$[TiF_xL_y]^{z-} \qquad (I)$$

dans laquelle L représente un composé de formule (II) suivante :

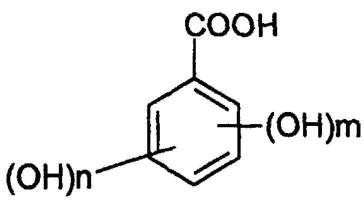

**(II)**

dans laquelle m est 0, 1 et n est 0, 1 ou 2,
et x représente 2, 4 ou 5, y représente 1 ou 2 et z représente 0, 1 ou 2; sous forme d'énantiomères, de diastéréoisomères, ainsi que leurs mélanges, y compris les mélanges racémiques, de bases libres ou de sels pharmaceutiquement acceptables, pour utilisation comme agent de protection contre la carie dentaire.

**Patentansprüche**

**1.** Von Titan abgeleitete Verbindung der folgenden Formel (I):

$$[TiF_xL_y]^{z-} \qquad (I)$$

in der L eine Verbindung der folgenden Formel (II) darstellt:

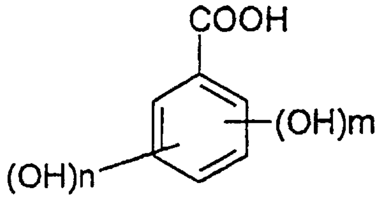

(II)

in der m 1 und n 0, 1 oder 2 bedeuten.
und x 2, 4 oder 5, y 1 oder 2 und z 0, 1 oder 2 bedeuten; in Form der Enantiomeren, der Diastereoisomeren sowie Mischungen davon einschließlich der racemischen Mischungen, der freien Basen oder der pharmazeutisch annehmbaren Salze.

2. Verbindung nach Anspruch 1, **dadurch gekennzeichnet, daß** die Verbindungen L ausgewählt sind aus Benzoesäurederivaten, insbesondere 2-Hydroxy-benzoesäure der Formel:

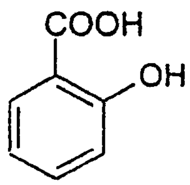

3-Hydroxy-benzoesäure der Formel:

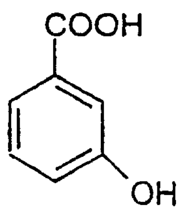

4-Hydroxybenzoesäure der Formel:

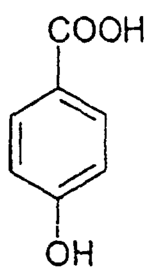

2,3-Dihydroxy-benzoesäure der Formel:

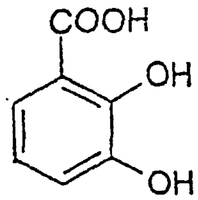

und 3,4-Dihydroxy-benzoesäure der Formel:

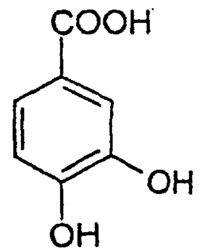

**3.** Verbindung nach Anspruch 1, **dadurch gekennzeichnet, daß** sie der folgenden Formel (III) entspricht:

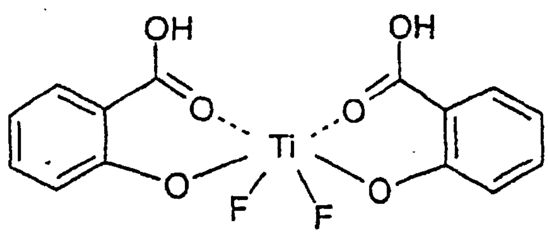

**(III)**

**4.** Verfahren zur Herstellung einer von Titan abgeleiteten Verbindung nach irgendeinem der Ansprüche 1 bis 3, **dadurch gekennzeichnet, daß** man festes Titan(IV)-fluorid mit einer Lösung der Benzoesäure in eine wasserfreien Lösungsmittel, wie Acetonitril, unter einer Stickstoffatmosphäre umsetzt.

**5.** Zubereitung zur bukkalen Anwendung zur Bekämpfung der Zahnkaries, **dadurch gekennzeichnet, daß** sie mindestens eine von Titan abgeleitete Verbindung der folgenden Formel (I) enthält:

$$[TiF_xL_y]^{z-} \quad (I)$$

in der L eine Verbindung der folgenden Formel (II) darstellt:

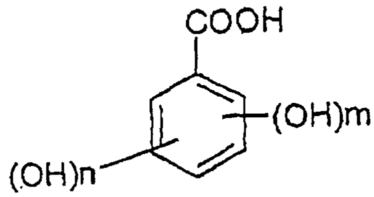

(II)

in der m 0, 1 und n 0, 1 oder 2 bedeuten,
und x 2, 4 oder 5, y 1 oder 2 und z 0, 1 oder 2 bedeuten; in Form der Enantiomeren, der Diastereoisomeren sowie Mischungen davon einschließlich der racemischen Mischungen, der freien Basen oder der pharmazeutisch annehmbaren Salze.

**6.** Zubereitung zur bukkalen Anwendung nach Anspruch 5, **dadurch gekennzeichnet, daß** sie mindestens eine von Titan abgeleitete Verbindung in einer Menge enthält, die etwa 10 ppm bis etwa 10 000 ppm Fluor äquivalent ist.

**7.** Zubereitung zur bukkalen Anwendung nach Anspruch 5 oder 6, **dadurch gekennzeichnet, daß** sie in Form einer Zahnpasta oder eines Zahnputzgels, einer Munddusche, eines Sprühpräparats, eines Schaums, eines Gurgelpräparats, eines Zahngels oder eines Kaugummis, einer Salbe, einer Paste, eines Lacks, von Pastillen, von Tabletten, einer Mundspülung, eines Pulvers oder einer gegebenenfalls konzentrierten Lösung vorliegt.

**8.** Zubereitung zur bukkalen Anwendung nach irgendeinem der Ansprüche 5 bis 7, **dadurch gekennzeichnet, daß** sie zusätzlich mindestens ein Poliermittel anorganischen oder organischen Ursprungs in Mengen von bis zu 80 Gew.-%, bezogen auf das Gesamtgewicht der Zubereitung, enthält.

**9.** Zubereitung zur bukkalen Anwendung nach Anspruch 8, **dadurch gekennzeichnet, daß** das Poliermittel insbesondere Calciumcarbonat, Calciumbicarbonat, Magnesiumcarbonat, Magnesiumbicarbonat, Natriumcarbonat, Natriumbicarbonat, Calciumphosphat, Calciumsulfat, Aluminiumoxid, Aluminiumoxid-hydrat, Siliciumdioxide, Oxide, Hydroxide, Trisilicate und Pyrophosphate von Magnesium, Celluloseverbindungen, die man durch Vermahlen von Getreidekörnern erhält, Natriummetaphosphat, Kaliummetaphosphat, Calciumphosphat-dihydrat, Dicalciumphosphat, Tricalciumphosphat. Calciumpyrophosphat, Aluminiumoxid, Aluminiumoxid-hydrate und insbesondere Aluminiumoxid-trihydrate, Aluminiumsilicate. Zirconiumsilicate, Bentonit, Magnesiumorthophosphat oder Trimagnesiumphosphat umfaßt.

**10.** Zubereitung zur bukkalen Anwendung nach irgendeinemd er Ansprüche 5 bis 9, **dadurch gekennzeichnet, daß** sie zusätzlich eines oder mehrere Bindemittel in Mengen von bis zu etwa 10 Gew.-%, bezogen auf das Gesamtgewicht der Zubereitung, enthält, insbesondere ausgewählt aus den natürlichen Verdickungsmitteln, wie Alginaten und Pectinen, natürlichen Gummen, wie Tragantgummi, Xanthangummi, Guargummi oder Caroubagummi oder Carragheenangummi, synthetische Carratheenate oder synthetische Verdickungsmittel, wie Cellulosederivate, wie Natriumcarboxymethylcellulose, Methylcellulose, Hydroxyalkylcellulosen oder vernetzte Polyacrylsäuren.

**11.** Zubereitung zur bukkalen Anwendung nach irgendeinem der Ansprüche 5 bis 10, **dadurch gekennzeichnet, daß** sie zusätzlich eines oder mehrere anionische, amphotere, zwitterionische, kationische oder nichtionische oberflächenaktive Mittel enthält.

**12.** Zubereitung zur bukkalen Anwendung nach irgendeinem der Ansprüche 5 bis 11, **dadurch gekennzeichnet, daß** sie zusätzlich einen oder mehrere in der Mundhygiene verwendete Wirkstoffe enthält, insbesondere die bekannten Mittel zur Verringerung des Mundgeruchs, wie beispielsweise Chlorhexidin. Cetylpyridiniumchlorid, Cyclodextrine oder Zinkverbindungen, wie Zinkhalogenide, Zinkacetat, Zinkcitrat oder Zinkfluorid.

**13.** Zubereitung zur bukkalen Anwendung nach irgendeinem der Ansprüche 5 bis 12, **dadurch gekennzeichnet, daß** sie zusätzlich ein oder mehrere Bindemittel, Verdickungsmittel, Antiobiotika. Süßungsmittel. Befeuchtungsmittel oder Erfrischungsmittel. Peptisierungsmittel, Konservierungsmittel. Zuckerstoffe, Farbstoffe, Aromastoffe, aromatisierende Substanzen und Mittel, Weichmacher, antibakterielle Mittel oder Bakterizide, Vitamine, Mittel gegen

Zahnbelag, vernarbende Mittel, vasomotorische Mittel, blutstillende Mittel, auf das Zahnfleisch einwirkende Mittel, antiinflammatorische Mittel, wie Enoxolon, Benzydamin, Allantoin und Permethol enthält.

**14.** Zubereitung zur bukkalen Anwendung nach Anspruch 13, **dadurch gekennzeichnet, daß** die Süßungsmittel Saccharose, Lactose, Fructose, Xylitol, Natriumcyclamat, Maltose, Natriumsaccharinat, Natriumglycyrrhizinat, Ammoniumglycyrrhizinat, alpha-Glucosyl/Steviolglucosid-Mischungen, D-Mannitol, Aspartam, Acesulfam K, Sorbitol, Lycosin und Mischungen davon umfassen.

**15.** Zubereitung zur bukkalen Anwendung nach Anspruch 13, **dadurch gekennzeichnet, daß** die antibakteriellen Mittel essentielle Öle, Pflanzenextrakte oder Substanzen, wie Alexidin, Octinidin, Hexetidin, Phenoxyethanol, Phenethylalkohol, Triclosan, Chlorhexidin, Cetylpyridiniumchlorid und Delmopinol in Mengen von bis zu etwa 10 Gew.-%, bezogen auf das Gesamtgewicht der Zubereitung, umfassen.

**16.** Von Titan abgeleitete Verbindung entsprechend der folgenden Formel (I):

$$[TiF_xL_y]^{z-} \qquad (I)$$

in der L eine Verbindung der folgenden Formel (II) darstellt:

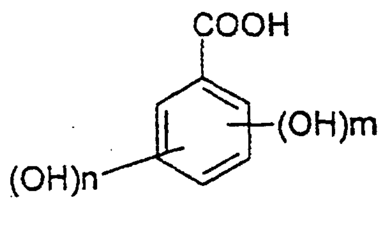

(II)

in der m 0 oder 1 und n 0, 1 oder 2
und x 2, 4 oder 5, y 1 oder 2 und z 0, 1 oder 2 bedeuten; in Form der Enantiomeren, der Diastereoisomeren sowie Mischungen davon, einschließlich der racemischen Mischungen, der freien Basen oder der pharmazeutisch annehmbaren Salze zur Verwendung als Mittel zum Schutz gegen Zahnkaries.

## Claims

**1.** Titanium-derived compound satisfying the formula (I) below:

$$[TiF_xL_y]^{z-} \qquad (I)$$

in which L represents a compound of formula (II) below:

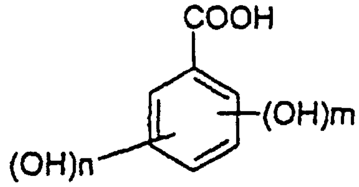

(II)

in which m is 1 and n is 0, 1 or 2,
and x represents 2, 4 or 5, y represents 1 or 2 and z represents 0, 1 or 2; in the form of enantiomers, of diastereoisomers, as well as the mixtures thereof, including the racemic mixtures, of free bases or of pharmaceutically acceptable salts.

**2.** Compound according to Claim 1, **characterized in that** the compounds L are chosen from benzoic acid derivatives, in particular 2-hydroxybenzoic acid of formula:

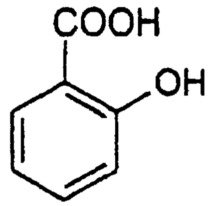

, 3-hydroxybenzoic acid of formula:

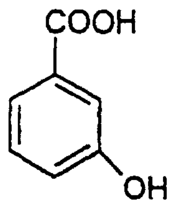

, 4-hydroxybenzoic acid of formula:

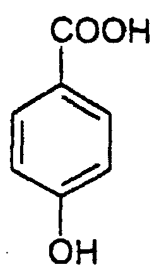

, 2,3-dihydroxybenzoic acid of formula:

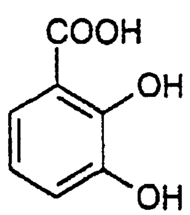

, 3,4-dihydroxybenzoic acid of formula:

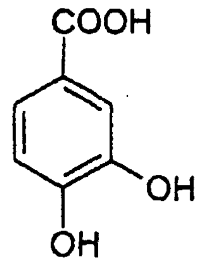

3. Compound according to Claim 1, **characterized in that** it satisfies the formula below (III) :

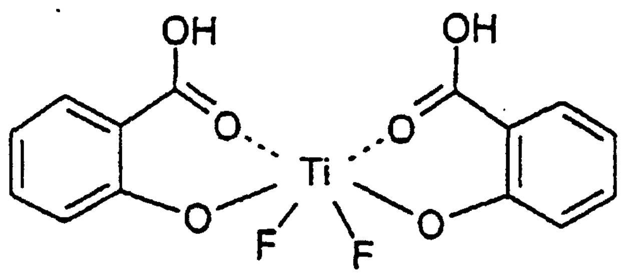

(III)

4. Method for preparing a titanium-derived compound according to any one of Claims 1 to 3, **characterized in that** solid titanium IV fluoride is reacted with a solution of benzoic acid in an anhydrous solvent such as acetonitrile, under a nitrogen atmosphere.

5. Composition for buccal use and intended to combat dental caries, **characterized in that** it comprises at least one titanium-derived compound satisfying the formula (I) below:

$$[TiF_xL_y]^{z-} \quad (I)$$

in which L represents a compound of formula (II) below:

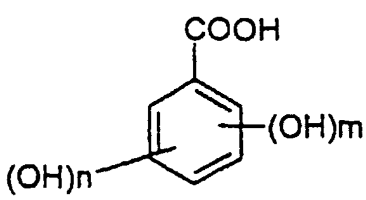

(II)

in which m is 0 or 1 and n is 0, 1 or 2,
and x represents 2, 4 or 5, y represents 1 or 2 and z represents 0, 1 or 2; in the form of enantiomers, of diastereoisomers, as well as the mixtures thereof, including the racemic mixtures, of free bases or of pharmaceutically acceptable salts.

6. Composition for buccal use according to Claim 5,
**characterized in that** it comprises at least one titanium-derived compound in an amount which is equivalent to

from approximately 10 ppm to approximately 10,000 ppm of fluorine.

7. Composition for buccal use according to either of Claims 5 and 6, **characterized in that** it is in the form of toothpaste or toothgel, of mouthwash, of spray, of foam, of gargling product, of dental gel or of chewing gum, balm, paste, glaze, lozenge, tablet, antiseptic throat preparation, powder, or concentrated or unconcentrated solution.

8. Composition for buccal use according to any one of Claims 5 to 7, **characterized in that** it also comprises at least one polishing agent of inorganic or organic origin in proportions ranging up to 80% by weight with respect to the total weight of the composition.

9. Composition for buccal use according to Claim 8, **characterized in that** the polishing agent comprises in particular calcium, magnesium or sodium carbonate or bicarbonate, calcium phosphates and sulphates, alumina and hydrated alumina, silicas, magnesium oxides, hydroxides, trisilicates and pyrophosphates, cellulose compounds obtained by crushing cereal seeds, sodium or potassium metaphosphates, calcium phosphate dihydrate, dicalcium phosphate, tricalcium phosphate, calcium pyrophosphate, alumina, hydrated, and in particular trihydrated, aluminas, aluminium or zirconium silicates, bentonite, as well as magnesium orthophosphate or trimagnesium phosphate.

10. Composition for buccal use according to any one of Claims 5 to 9, **characterized in that** it also comprises one or more cohesion agents, in proportions ranging up to approximately 10% by weight with respect to the total weight of the composition, chosen in particular from natural thickeners such as alginates or pectins, natural gums such as gum tragacanth or xanthan, guar, carob or carrageenan gums, synthetic carrageenates, and synthetic thickeners such as cellulose derivatives, for instance the sodium salt of carboxymethylcellulose, methylcellulose, hydroxyalkylcelluloses or crosslinked polyacrylic acids.

11. Composition for buccal use according to any one of Claims 5 to 10, **characterized in that** it also comprises one or more surfactants of anionic, amphoteric, zwitterionic, cationic or nonionic nature.

12. Composition for buccal use according to any one of Claims 5 to 11, **characterized in that** it also comprises one or more active agents used in buccal hygiene, in particular agents known to reduce bad breath, such as for example chlorhexedine, cetylpyridinium chloride, cyclodextrins or zinc compounds such as zinc halides, zinc acetate, zinc citrate or zinc fluoride.

13. Composition for buccal use according to any one of Claims 5 to 12, **characterized in that** it also comprises one or more cohesion agents, thickeners, antibiotics, sweetening, wetting or refreshing agents, peptizing agents, preserving agents, sweeteners, dyes, aromas, flavourings and flavour-enhancing substances, plasticizers, antibacterial agents or bactericides, vitamins, antitartar agents, healing agents, vasomotor agents, anti-bleeding agents, agents which are active on the gums, anti-inflammatory agents such as enoxolone, benzydamine, allantoin or permethol.

14. Compositions for buccal use according to Claim 13, **characterized in that** the sweetening agents comprise sucrose, lactose, fructose, xylitol, sodium cyclamate, sodium saccharinate or maltose, sodium or ammonium glycyrrhizinates, alpha-glucosyl/ steviolglucoside mixtures, D-mannitol, aspartame, acesulfame K, sorbitol, lycosin and mixtures thereof.

15. Composition for buccal use according to Claim 13, **characterized in that** the antibacterial agents comprise essential oils, plant extracts or substances such as alexidine, octinidine, hexetidine, phenoxyethanol, phenethyl alcohol, triclosan, chlorhexidine, cetylpyridinium chloride and delmopinol, in proportions ranging up to approximately 10% by weight with respect to the total weight of the composition.

16. Titanium-derived compound satisfying the formula (I) below:

$$[TiF_xL_y]^{z-} \qquad (I)$$

in which L represents a compound of formula (II) below:

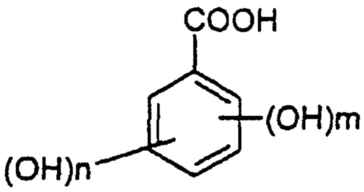

(II)

in which m is 0 or 1 and n is 0, 1 or 2,
and x represents 2, 4 or 5, y represents 1 or 2 and z represents 0, 1 or 2; in the form of enantiomers, of diastereoisomers, as well as the mixtures thereof, including the racemic mixtures, of free bases or of pharmaceutically acceptable salts, for use as protecting agent against dental caries.